# EUROPÄISCHE PATENTANMELDUNG

(11) **EP 1 733 677 A1**
(43) Veröffentlichungstag der Anmeldung: **20.12.2006**
(21) Anmeldenummer: 05013177.0
(22) Anmeldetag: 18.06.2005
(51) Int. Cl.: A61B 5/00

(54) **Blutzuckermessgerät mit Signaleinrichtung**

(71) Anmelder: Roche Diagnostics GmbH, 68305 Mannheim (DE); F. HOFFMANN-LA ROCHE AG, 4070 Basel (CH)
(72) Erfinder: Werner, Karl, 69168 Wiesloch (DE); Stephan, Peter, 68167 Mannheim (DE); Lorenz, Robert, 67547 Worms (DE)
(74) Vertreter: Pfeifer, Hans-Peter

(57) **Zusammenfassung**

Die Erfindung betrifft ein Blutzuckermeßgerät zur Ermittlung der Blutglucosekonzentration von Diabetikern, umfassend eine Meßeinrichtung (6) zum Ermitteln eines Blutglucosekonzentrationswertes, eine Anzeigeeinrichtung (4) zum Anzeigen des Blutglucosekonzentrationswertes, eine Zeitmeßeinrichtung (9), eine Signaleinrichtung (10), um zu festgelegten Tageszeiten ein Erinnerungssignal zu erzeugen, einen Speicher (8) und eine Steuer- und Auswerteeinrichtung (7) zum Betätigen der Signaleinrichtung (10) Erfindungsgemäß ist vorgesehen, daß die Steuer- und Auswerteeinrichtung (7) so ausgebildet ist, daß von ihr auf der Grundlage von in einem Speicher (8) gespeicherten Daten über das bisherige Benutzerverhalten ein individuelles Benutzerprofil ermittelt wird, gemäß welchem von der Steuer- und Auswerteeinrichtung (7) Erinnerungszeiten ermittelt werden, zu denen die Signaleinrichtung (10) von der Steuer- und Auswerteeinrichtung (7) betätigt wird.

## Beschreibung

Die Erfindung betrifft ein Blutzuckermeßgerät zur Ermittlung der Blutglucosekonzentration von Diabetikern, umfassend eine Meßeinrichtung zum Ermitteln von Blutglucosekonzentrationwerten, eine Anzeigeeinrichtung zum Anzeigen von Blutglucosekonzentrationwerten, eine Signaleinrichtung zum Erzeugen eines Erinnerungssignals und eine Steuer- und Auswerteeinrichtung zum Betätigen der Signaleinrichtung.

Derartige Blutzuckermeßgeräte sind beispielsweise unter der Marke "Accu-Check Go" im Handel erhältlich. Die Signaleinrichtung des vorbekannten Blutzuckermeßgeräts Accu-Check Go kann bis zu drei Tageszeiten speichern, zu denen der Benutzer durch einen Signalton an eine anstehende Blutzuckermessung erinnert wird. Die entsprechenden Tageszeiten werden von dem Benutzer gewählt und über Tasten in das Gerät eingegeben.

Da Blutzuckermeßgeräte von Diabetikern ständig mitgeführt werden müssen, besteht die Forderung, diese möglichst kompakt zu gestalten. Dies hat jedoch zur Folge, daß Bedienungselemente wie Tasten oder Ähnliches nur in sehr kleiner Form und einer geringen Anzahl zur Verfügung gestellt werden können. Das Einprogrammieren von Tageszeiten, zu denen durch einen Signalton an eine Blutzuckermessung erinnert werden soll, ist deshalb für viele Benutzer sehr mühsam. Dies trifft insbesondere auf Personen zu, deren manuelle Geschicklichkeit durch Alter oder Krankheit eingeschränkt ist.

Aufgabe der Erfindung ist es deshalb, einen Weg aufzuzeigen, wie es Benutzern von Blutzuckermeßgeräten erleichtert werden kann, zu individuell festgelegten Tageszeiten an das Durchführen einer Blutzuckermessung erinnert zu werden.

Diese Aufgabe wird mit einem Blutzuckermeßgerät der eingangs genannten Art erfindungsgemäß dadurch gelöst, daß die Steuer- und Auswerteeinrichtung so ausgebildet ist, daß von ihr auf der Grundlage von in einem Speicher gespeicherten Daten über das bisherige Benutzerverhalten ein individuelles Benutzerprofil ermittelt wird, gemäß welchem von der Steuer- und Auswerteeinrichtung Erinnerungszeiten ermittelt werden, zu denen die Signaleinrichtung von der Steuer- und Auswerteeinrichtung betätigt wird.

Bei einem erfindungsgemäßen Blutzuckermeßgerät ist es deshalb nicht mehr erforderlich, daß irgendwelche Tageszeiten von einem Benutzer einprogrammiert werden. Aus gespeicherten Daten über das bisherige Benutzerverhalten ermittelt die Auswerteeinrichtung selbsttätig die Tageszeiten, zu denen von der Signaleinrichtung ein Erinnerungssignal erzeugt wird. Die Erinnerungsfunktion des Geräts paßt sich also von selbst an den individuellen Tagesablauf und das Verhalten eines Benutzers an. Besonders vorteilhaft ist dabei, daß sich die Erinnerungsfunktion selbsttätig an eine Änderung des Tagesrhythmus anpaßt, die beispielsweise jahreszeitlich bedingt sein kann. Die Erinnerungsfunktion eines erfindungsgemäßen Geräts stellt deshalb ein selbstlernendes Alarmsystem dar, so daß der Benutzer automatisch an zur Behandlung von Diabetes wichtige Maßnahmen, beispielsweise Messungen der Blutglucosekonzentration, erinnert wird.

Die Erinnerungszeiten, zu denen die Signaleinrichtung betätigt wird, können beispielsweise durch Anwendung eines Mustererkennungsverfahrens ermittelt werden. Bei den Erinnerungszeiten handelt es sich bevorzugt um Tageszeiten, zu denen der Benutzer beispielsweise gewohnheitsmäßig Messungen der Blutglucosekonzentration vornimmt. Die Erinnerungszeiten können aber auch von vordefinierten Ereignissen gemäß dem ermittelten Benutzerprofil abhängen. Beispielsweise kann dem Gerät die Einnahme einer Mahlzeit durch Tastendruck mitgeteilt werden und nach einem von dem bisherigen Meßverhalten abhängigen Zeitintervall an eine Messung erinnert werden. Je nach der Art des vordefinierten Ereignisses und den gespeicherten Daten über das bisherige Benutzerverhalten kann dieses Zeitintervall unterschiedlich lang, im Extremfall sogar Null, sein.

Die gespeicherten Daten über das Benutzerverhalten enthalten bevorzugt Ereigniszeitpunkte, deren Speicherung von der Steuer- und Auswerteeinheit bei Eintritt eines im voraus definierten Ereignisses als ein die jeweils aktuelle Tageszeit charakterisierendes Signal veranlaßt wird. Zum Ermitteln der Erinnerungszeiten werden mittels der Steuer- und Auswerteeinrichtung mindestens ein Teil der gespeicherten Ereigniszeitpunkte ausgewertet, wobei die für die Auswertung verwendeten Ereigniszeitpunkte über mehrere Tage verteilt sind.

Bei den gespeicherten Ereigniszeitpunkten handelt es sich bevorzugt um Zeitpunkte, zu denen Blutglucosekonzentrationswerte gemessen wurden, so daß ein Benutzer durch ein von der Signaleinrichtung erzeugtes Erinnerungssignal an das Durchführen einer Messung der Blutglucosekonzentration erinnert wird. Von der Steuer- und Auswerteeinheit können jedoch auch andere Ereignisse, beispielsweise das Einnehmen einer Mahlzeit oder das Verabreichen einer Insulingabe beim Ermitteln einer Erinnerungszeit berücksichtigt werden. Derartige Ereignisse können der Steuer-und Auswerteeinrichtung durch Betätigung eines Bedienungselements, beispielsweise durch Tastendruck, von dem Benutzer mitgeteilt werden.

Auf diese Weise können Erinnerungszeitpunkte ermittelt werden, die individuell an den Tagesrhythmus eines Benutzers angepaßt sind und dennoch auch von Ereignissen, die für den zeitlichen Verlauf der Blutglucosekonzentration wichtig sind, beispielsweise Mahlzeiten, Insulingaben oder körperliche Betätigung, abhängen.

Das Auswerten von derartigen Ereigniszeitpunkten (Mahlzeiten, Insulingaben, sportliche Betätigung) ermöglicht es zudem, Erinnerungssignale zu erzeugen, mit denen an Insulingaben und/oder Mahlzeiten erinnert wird. Besonders günstig ist dabei, wenn mit der Signaleinrichtung unterschiedliche Erinnerungssignale erzeugt werden können.

Beispielsweise kann von der Steuer- und Auswerteeinrichtung eine erste Klasse von Erinnerungszeiten ermittelt werden, zu denen die Signaleinrichtung ein Erinnerungssignal eines ersten Typs erzeugt, um an eine Messung der Blutglucosekonzentrationzu erinnern, und zusätzlich eine zweite Klasse von Erinnerungszeiten ermittelt werden, zu denen von der Signaleinrichtung ein Erinnerungssignal eines zweiten Typs erzeugt wird, um an Insulin, Mahlzeiten und/oder sportliche Betätigung zu erinnern.

Die Erfindung wird nachfolgend anhand eines Ausführungsbeispiels unter Bezugnahme auf die beigefügten Zeichnungen näher erläutert. Die darin dargestellten Besonderheiten können einzeln oder in Kombination verwendet werden, um bevorzugte Ausgestaltungen zu schaffen. Es zeigen:
- Fig. 1: eine perspektivische Ansicht eines Blutzuckermeßgeräts und
- Fig. 2: ein Blockschaltbild des in Figur 1 gezeigten Blutzuckermeßgeräts.

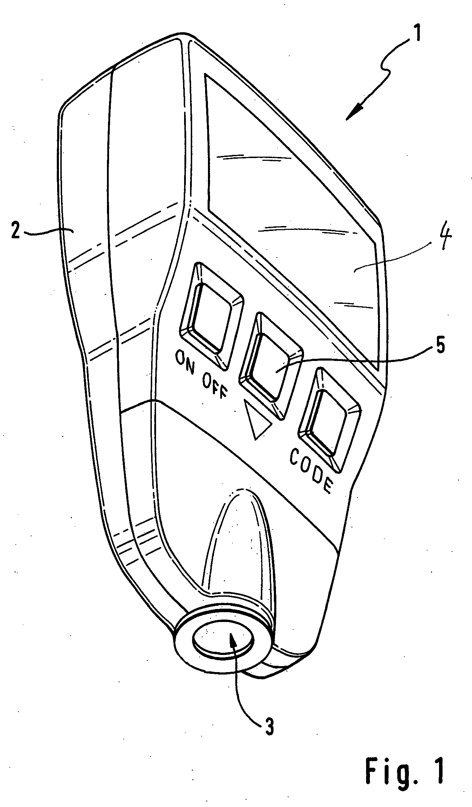

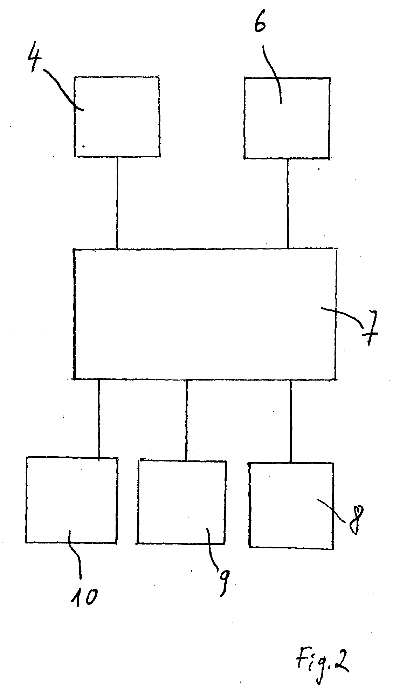

Das in Figur 1 gezeigte Blutzuckermeßgerät 1 entspricht in seiner äußeren Erscheinung bekannten Geräten. Zum Anzeigen von Meßwerten dient eine Anzeigeeinrichtung 4 in Form eines Displays. Zum Betätigen des Meßgeräts 1 und/oder zum Benutzen von Sonderfunktionen sind Bedienungselemente 5 in Form von Tasten vorhanden. In dem Gerät befindet sich eine Lanzette (nicht dargestellt), mit der in einem Körperteil, beispielsweise einem Finger, der an eine Öffnung 3 des Gehäuses 2 angelegt wird, eine Einstichwunde erzeugt werden kann: Das Gerät 1 nimmt selbsttätig aus der Einstichwunde austretendes Blut auf und ermittelt mit einer Meßeinrichtung (siehe Figur 2) einen Blutglucosekonzentrationswert.

Das Blutzuckermeßgerät 1 ist mit einer netzunabhängigen Stromquelle (nicht dargestellt), beispielsweise handelsüblichen Batterien, ausgerüstet, mit der die einzelnen Komponenten gespeist werden. Die entsprechenden Komponenten des Blutzuckermeßgeräts 1 sind bekannt und bedürfen deshalb keiner näheren Erläuterung.

In Figur 2 ist schematisch ein Blockschaltbild des Blutzuckermeßgeräts 1 dargestellt. Der Blutzuckergehalt' einer Blutprobe wird mit einer Meßeinrichtung 6, die an eine Steuer- und Auswerteeinrichtung 7 angeschlossen ist, ermittelt. Die Steuer- und Auswerteeinrichtung 7 enthält einen Mikroprozessor. Ermittelte Blutglucosekonzentrationswerte werden in einem nichtflüchtigen Speicher 8 gespeichert und mit der Anzeigeeinrichtung 4 einem Benutzer angezeigt.

Das Blutzuckermeßgerät 1 verfügt ferner über eine interne Zeitmeßeinrichtung 9, beispielsweise als Taktgeber der Steuer- und Auswerteeinrichtung 7, und eine Signaleinrichtung 10, um einen Benutzer zu festgelegten Tageszeiten, beispielsweise durch einen Signalton, an das Durchführen einer Blutglucosemessung zu erinnern.

In dem Speicher 8 werden als Ereigniszeitpunkte zusätzlich die Meßzeitpunkte, d.h. die Uhrzeiten, gespeichert, zu denen Blutglucosekonzentrationswerte gemessen wurden. Von der Steuer- und Auswerteeinrichtung 7 werden auf der Grundlage der gespeicherten Ereigniszeitpunkte Erinnerungszeiten ermittelt, zu denen die Signaleinrichtung 10 von der Steuer- und Auswerteeinrichtung 7 betätigt wird. Diese Erinnerungszeiten werden von der Steuer- und Auswerteeinrichtung 7 mit einem Verfahren ermittelt, das im Folgenden erläutert wird.

Bei der erstmaligen Inbetriebnahme des Blutzuckermeßgeräts 1 sind noch keine Ereigniszeitpunkte, d.h. Tageszeiten, zu denen Blutglucosemessungen durchgeführt wurden, gespeichert. Die Steuer- und Auswerteeinrichtung 7 beginnt deshalb mit ihrer eigentlichen Tätigkeit erst an dem zweiten Tag nach der erstmaligen Inbetriebnahme und greift dabei auf die gespeicherten Ereigniszeitpunkte zurück, zu denen an dem ersten Tag Blutglucosemessungen durchgeführt wurden. An dem zweiten Betriebstag werden die gespeicherten Ereigniszeitpunkte des ersten Betriebstags als Erinnerungszeiten verwendet. Die Signaleinrichtung 10 wird deshalb an dem zweiten Betriebstag von der Steuer- und Auswerteeinrichtung 7 genau zu jenen Tageszeiten betätigt, zu denen an dem ersten Betriebstag Blutglucosemessungen durchgeführt wurden.

Für den dritten und jeden weiteren Betriebstag werden die Erinnerungszeiten jeweils neu berechnet. Dazu werden jene Ereigniszeitpunkte betrachtet, die in einem vorgegebenen Zeitintervall um die zuletzt verwendete Erinnerungszeit liegen. Aus diesen Ereigniszeitpunkten wird die neue Erinnerungszeit als Mittelwert berechnet.

Die Größe dieses Zeitintervalls ist so zu wählen, daß beispielsweise Ereigniszeitpunkte der ersten Messung eines Tages bei der Berechnung der Erinnerungszeit für die zweite Messung des gleichen Tages unberücksichtigt bleiben. Das Zeitintervall sollte also so klein sein, daß in ihm zur Berechnung der Erinnerungszeit für die n-te Messung eines Tages nur Ereigniszeitpunkte von n-ten Messungen vergangener Tage enthalten sind. Bevorzugt umfaßt das Zeitintervall etwa ein bis zwei Stunden.

Auf diese Weise kann das Gerät 1 selbsttätig Erinnerungszeiten ermitteln, die dem individuellen Lebensrhythmus eines Benutzers entsprechen und an dessen Mahl- und Ruhezeiten angepaßt sind.

Bevorzugt werden bei der Berechnung der Erinnerungszeiten nur Werte eines begrenzten Zeitraums von beispielsweise zwei Wochen berücksichtigt, so daß eine Anpassung an einen geänderten Tagesrhythmus in überschaubarer Zeit erfolgt. Je kürzer dieser Zeitraum gewählt wird, desto schneller entsprechen die Erinnerungszeiten geänderten Lebensgewohnheiten. Dennoch darf dieser Zeitraum nicht zu kurz gewählt werden, da sonst Messungen, die aus besonderem Anlaß zu ungewohnten Zeiten durchgeführt wurden, einen unerwünscht großen Einfluß auf die Berechnung der Erinnerungszeiten haben. Günstig ist es, bei der Berechnung der Erinnerungszeiten jeweils die Ereigniszeitpunkte der letzten 4 bis 14 Tage zu verwenden.

In diesem Zusammenhang ist es vorteilhaft, wenn der Benutzer die Möglichkeit hat, gespeicherte Meßzeiten zu löschen oder für den ersten Tag Erinnerungszeiten einzuprogrammieren. Zu diesem Zweck kann die Anzeigeeinrichtung 4 zum Abbilden eines Bedienungsmenüs genutzt werden, aus dem mit den Bedienungselementen 5 geeignete Funktionen ausgewählt werden.

Bevorzugt bleiben bei der Ermittlung der Erinnerungszeiten Wiederholungsmessungen unberücksichtigt, die beispielsweise durchgeführt wurden, um auffällige Meßwerte mit besonders hohen oder niedrigen Blutglucosekonzentrationswerten zu überprüfen. In diesem Zusammenhang ist es bevorzugt, daß die Steuer- und Auswerteeinrichtung 7 jeweils ermittelte Blutglucosekonzentrationswerte mit historischen Meßwerten vergleicht und bei auffälligen Meßwerten nach einer vorgegebenen Zeit von beispielsweise 20 Minuten selbsttätig an eine Wiederholungsmessung erinnert.

Ein erhöhter Benutzerkomfort läßt sich dadurch erreichen, daß für Arbeitstage andere Erinnerungszeiten ermittelt werden als für Urlaubstage. Dies liegt daran, daß die meisten Menschen an Arbeitstagen einen anderen Tagesrhythmus haben als an Urlaubstagen. In diesem Zusammenhang sind unter Urlaubstagen alle arbeitsfreien Tage eines Benutzers zu verstehen, also auch Feiertage und Wochenenden, an denen der Benutzer nicht arbeitet. Am einfachsten können unterschiedliche Erinnerungszeiten für Arbeits- und Urlaubstage dadurch ermittelt werden, daß der Benutzer durch einen Tastendruck dem Gerät 1 mitteilt, ob der heutige Tag ein Urlaubstag ist.

Bevorzugt wird die Signaleinrichtung 10 zusätzlich dazu verwendet, um einen Benutzer zu festgelegten Tageszeiten an eine Basalinjektion, d.h. eine Insulininjektion zur Deckung seines Grundbedarfs, zu erinnern. Die entsprechenden Erinnerungszeiten für Basalinjektionen können ebenso wie die Erinnerungszeiten für Blutglucosemessungen mit dem beschriebenen Verfahren ermittelt werden. Dazu ist es lediglich erforderlich, daß dem Gerät der Zeitpunkt einer Basalinjektion mitgeteilt wird. Im einfachsten Fall kann dies durch einen Tastendruck geschehen, so daß in dem Speicher 8 die aktuelle Zeit zum Zeitpunkt des Tastendrucks als Ereigniszeitpunkt für eine Basalinjektion gespeichert wird. Bevorzugt wird mit der Signaleinrichtung 10 zum Erinnern an eine Blutglucosemessung ein anderes Signal als zum Erinnern an eine Basalinjektion erzeugt. Für das menschliche Ohr leicht unterscheidbare Erinnerungsignale können beispielsweise durch unterschiedliche Tonhöhen oder Tonfolgen erzeugt werden.

Mit der Steuer- und Auswerteeinrichtung 7 kann durch Auswerten von in dem Speicher 8 gespeicherten Meßwerten der Blutglucosekonzentration unter Berücksichtigung zusätzlicher Daten über Insulingaben, Mahlzeiten und körperliche Aktivitäten auch automatisch an Korrekturinsulingaben, Mahlzeiten oder körperliche Aktivitäten erinnert werden.

## Patentansprüche

1. Blutzuckermeßgerät zur Ermittlung der Blutglucosekonzentration von Diabetikern, umfassend:
eine Meßeinrichtung (6) zum Ermitteln von Blutglucosekonzentrationswerten,
eine Anzeigeeinrichtung (4) zum Anzeigen von Blutglucosekonzentrationswerten,
eine Signaleinrichtung (10) zum Erzeugen eines Erinnerungssignals und
eine Steuer- und Auswerteeinrichtung (7) zum Betätigen der Signaleinrichtung (10),
**dadurch gekennzeichnet, daß**
die Steuer- und Auswerteeinrichtung (7) so ausgebildet ist, daß von ihr auf der Grundlage von in einem Speicher (8) gespeicherten Daten über das bisherige Benutzerverhalten ein individuelles Benutzerprofil ermittelt wird, gemäß welchem von der Steuer- und Auswerteeinrichtung (7) Erinnerungszeiten ermittelt werden, zu denen die Signaleinrichtung (10) von der Steuer- und Auswerteeinrichtung (7) betätigt wird.

2. Blutzuckermeßgerät nach Anspruch 1, **dadurch gekennzeichnet, daß** die gespeicherten Daten über das Benutzerverhalten Ereigniszeitpunkte enthalten, deren Speicherung von der Steuer- und Auswerteeinheit (7) bei Eintritt eines im voraus definierten Ereignisses als ein die jeweils aktuelle Tageszeit charakterisierendes Signal veranlaßt wurde, und
zum Ermitteln der Erinnerungszeiten mittels der Steuer- und Auswerteeinrichtung (7) mindestens ein Teil der gespeicherten Ereigniszeitpunkte ausgewertet wird, wobei die für die Auswertung verwendeten Ereigniszeitpunkte über mehrere Tage verteilt sind.

3. Blutzuckermeßgerät nach Anspruch 2, **dadurch gekennzeichnet, daß** das im voraus definierte Ereignis eine Messung der Blutglucosekonzentration ist.

4. Blutzuckermeßgerät nach Anspruch 3, **dadurch gekennzeichnet, daß** die Steuer- und Auswerteeinrichtung (7) so eingerichtet ist, daß das Betätigen der Signaleinrichtung (10) zu einer ermittelten Erinnerungszeit unterbleibt, wenn in einem vorgegebenen Karenzzeitintervall von vorzugsweise 20 Minuten vor der Erinnerungszeit eine Messung der Blutglucosekonzentration durchgeführt wurde.

5. Blutzuckermeßgerät nach einem der vorhergehenden Ansprüche, **dadurch gekennzeichnet, daß** das im voraus definierte Ereignis das Betätigen eines Bedienungselements (5) des Blutzuckermeßgeräts (1) durch den Benutzer ist.

6. Blutzuckermeßgerät nach einem der vorhergehenden Ansprüche, **dadurch gekennzeichnet, daß** die Erinnerungszeiten mittels eines Mustererkennungsverfahrens ermittelt werden.

7. Blutzuckermeßgerät nach einem der vorhergehenden Ansprüche, **dadurch gekennzeichnet, daß** für Arbeitstage andere Erinnerungszeiten ermittelt werden als für arbeitsfreie Tage.

8. Blutzuckermeßgerät nach einem der vorhergehenden Ansprüche, **dadurch gekennzeichnet, daß** der Speicher (8) ein nichtflüchtiger Speicher (8) ist.

9. Blutzuckermeßgerät nach einem der vorhergehenden Ansprüche, **dadurch gekennzeichnet, daß** von der Signaleinrichtung (10) akustische Erinnerungssignale erzeugt werden.

10. Blutzuckermeßgerät nach einem der vorhergehenden Ansprüche, **dadurch gekennzeichnet, daß** von der Steuer-und Auswerteeinrichtung (7) eine erste Klasse von Erinnerungszeiten ermittelt wird, zu denen von der Signaleinrichtung (10) ein Erinnerungssignal eines ersten Typs erzeugt wird, um an eine Messung der Blutglucosekonzentration zu erinnern, und
von der Steuer- und Auswerteeinrichtung (7) eine zweite Klasse von Erinnerungszeiten ermittelt wird, zu denen von der Signaleinrichtung (10) ein Erinnerungssignal eines zweiten Typs erzeugt wird, um an eine Insulininjektion zu erinnern.
